# EUROPEAN PATENT APPLICATION

(11) **EP 4 768 476 A1**
(43) Date of publication of application: **01.07.2026**
(21) Application number: 24315601.5
(22) Date of filing: 24.12.2024
(51) Int. Cl.: C07D 401/04

(54) **USE OF A MECHANICAL FORCE IN THE IMPLEMENTATION OF A PROCESS OF PREPARATION OF IMMUNOMODULATORY DRUGS**

(71) Applicant: UNIVERSITE DE MONTPELLIER, 34090 Montpellier (FR); Centre National de la Recherche Scientifique, 75016 Paris (FR); Ecole Nationale Supérieure de Chimie de Montpellier, 34296 Montpellier Cedex 5 (FR)
(72) Inventor: Chatzigiannis, Christos, 34090 Montpellier (FR); Solorzano-Rodriguez, Ruben, 34170 Casteinau-le-Lez (FR); Colacino, Evelina, 34090 Montpellier (FR); Bordier, Corentin, 72120 Saint-Calais (FR)
(74) Representative: Cabinet Grosset-Fournier & Demachy

(57) **Abstract**

The invention relates to the use of a mechanical force to generate a mechanochemical reaction in the implementation of a process of preparation of immunomodulatory drugs of Formula (I) or a pharmaceutically acceptable salt thereof, and their process of preparation.

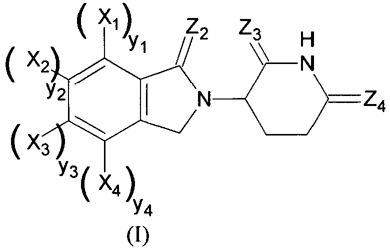

## Description

The present invention refers to the use of a mechanical force to generate a mechanochemical reaction in the implementation of a process of preparation of immunomodulatory drugs, and their process of preparation.

### Background of the Invention

Over the past century, intensified human activities involving chemistry have led to significant environmental issues, including ozone layer depletion, global warming, air pollution, and overexploitation of natural resources. In response, several various measures have been implemented over the last 50 years to mitigate the negative environmental impacts of chemical production.

Acknowledging the necessity to lessen the environmental damage caused by the chemical industry has spurred the growth of mechanochemistry.

This approach seeks to safeguard future generations by advancing more sustainable chemical processes. Mechanochemistry allows reactions to occur without solvents or with minimal liquid media, in a process named Liquid Assisted Grinding (LAG), optionally with a solid additive, making it a viable alternative for green chemical synthesis. Its advantages include the absence of bulk solvents, increased kinetics, precise stoichiometric control (using reagents in exact amounts rather than excess), and more selective reactions, which simplify work-up procedures, thus reducing the amount of total waste generated ('Fantozzi, N.; Volle, J-N., Porcheddu, A., Virieux D., Garcia, F., Colacino, E. "Green metrics in mechanochemistry" Chem. Soc. Rev. 2023, 52, 6680-6714. DOI: 10.1039/D2CS00997H' and 'Galant, O.; Cerfeda, G.; McCalmont, A.S.; James, S.L.; Porcheddu, A.; Delogu, F.; Crawford, D.E.; Colacino, E.; Spatari, S. "Mechanochemistry can reduce life cycle environmental impacts of manufacturing active pharmaceutical ingredients ACS Sustainable Chem. Eng. 2022, 10, 1430-1439 https://doi.org/10.1021/acssuschemeng.1c06434'). These features highlight the superiority of mechanochemical processes compared to traditional solution-based chemistry.

Lenalidomide and its derivatives are highly effective immunomodulatory drug (IMiD) used to treat several hematological cancers, including multiple myeloma, mantle cell lymphoma, chronic lymphocytic leukemia, and myelodysplastic syndrome with chromosome 5q deletion syndrome. It acts as a molecular glue, enhancing the degradation of specific neosubstrates by recruiting them to the E3 ubiquitin ligase CRL4CRBN complex through its interaction with cereblon (CRBN). This mechanism leads to the targeted elimination of proteins like IKZF1, IKZF3, and CK1α, which are crucial for its anti-cancer effects. Lenalidomide and its derivatives also enhances immune function by stimulating IL-2 production in T lymphocytes and reducing pro-inflammatory cytokines. Its dual action on protein degradation and immune modulation has established it as a cornerstone therapy for various hematological malignancies, significantly improving patient outcomes.

One of the aims of the invention is to provide a new use of a mechanical force to conduct a mechanochemical total synthesis to provide lenalidomide or a pharmaceutically acceptable salt thereof.

Another aim of the invention is to provide a mechanochemical process of preparation of lenalidomide or a pharmaceutically acceptable salt thereof.

Additional aim of the invention is the reduction of the amount of starting and intermediate materials in comparison with the manufacturing method in solution.

Another additional aim of the invention is the reduction of the amount of the energy for the heating to carry out the chemical process, and for the cooling which results from it in comparison with the manufacturing method in solution.

Another additional aim of the invention is the short reaction time and the fact that the process is carried out in devices operating at room temperature (20-22°C) in comparison with the manufacturing method in solution.

Another additional aim of the invention is the work-up that are easy to implement (no chromatography needed), because of the chemical transformation that is total.

### Brief summary of the invention

The Applicant has found that a mechanical force to generate a mechanochemical reaction in the implementation of a process of preparation of a compound of formula (I) can be used. Inventors were able to carry out a mechanochemical total synthesis of a compound of formula (I) in 2 steps without the use of metal catalysts (with precious metal(s) or not), by implementing mechanochemical processes for its preparation in 2 steps, the solution-based manufacturing method needs heating and precious metal to carry out the process.

### Detailed description of the invention

In a general embodiment, the invention relates to the use of a mechanical force to generate a mechanochemical reaction in the implementation of a process of preparation in a reactor of a compound of formula (I): 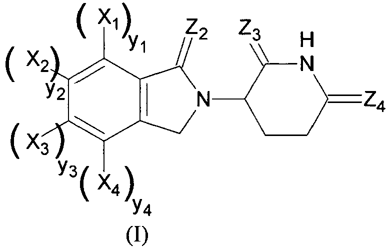 wherein:
- Z₂, Z₃ and Z₄, identical or different, are chosen among: oxygen or sulfur atom,
- X₁, X₂, X₃ and X₄, identical or different, are chosen among: (C₁-C₅)alkyl group, (C₁-C₅)alkoxy group, amino group, halogen atom, trifluoromethyl group, hydroxy group, (H or C₁-C₁₀)acyl group, a 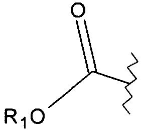 group, or a 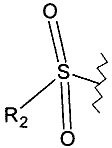 group
   wherein R₁ is chosen among: hydrogen atom, (C₁-C₅)alkyl group, or -NR₁ₐR_{1b} group, R₁ₐ and R_{1b}, identical or different, are chosen among: hydrogen atom or (C₁-C₅)alkyl group,
   and R₂ is chosen among: hydroxy group, (C₁-C₅)alkoxy group, -NR₂ₐR_{2b},
   R₂ₐ and R_{2b}, identical or different, are chosen among: hydrogen atom or (C₁-C₅)alkyl group
- y₁, y₂, y₃ and y₄, identical or different, are equal to 0 or 1, and provided that y₁ + y₂ + y₃ + y₄ is 1 or 2,
- and provided that at least one X₁, X₂, X₃ and X₄ is an amino group,

or a pharmaceutically acceptable salt thereof,
said process comprising 2 steps, using 3-aminopiperidine-2,6-dione hydrochloride as starting material, wherein at least one step is mechanochemical.

As used herein the expression "mechanochemical force" refers to mechanochemical activation or mechanochemistry, that is a technology and a research field in which solid chemical reactants, i.e in the form of granules or powders (pulverulent) or in the pasty state, are milled or ground together or ground together under mechanical activation and the chemical reactions are induced by the direct absorption of mechanical energy, (McNaught, A.D.; Wilkinson, A. IUPAC Compendium of Chemical Terminology ("The Golden Book"); 2nd edition; Blackwell Scientific publications, Oxford, 1997) without solvents (neat grinding) or with minimum/small amounts of solvent in a process named liquid-assisted grinding (LAG) in comparison with solution-based methods. The mechanochemical reaction occurs because of the activation by the mechanical force.

As used herein, the term "liquid-assisted grinding (LAG)" refers to minimum or limited amounts of a liquid, the role of which is to increase ease of mixing and/or to participate to the reaction occurring in a mechanochemical system. Under conventionally defined LAG conditions; the liquid additive has also been described as functioning essentially as a catalyst or lubrificant.

LAG uses a small amount of a liquid to accelerate reactions, as well as to enable transformations that do not take place by neat grinding. The empirical definition of LAG is based on the way mechanochemical reactivity is affected by the ratio of the liquid additive to the weight of chemical reactants (η, eta).

A value of η = 0 µL/mg corresponds to neat grinding, η > 2 µL/mg corresponds to a typical solution reaction, while LAG lies in the range of ≈0-1 µL/mg. In that range, reactivity appears independent of reactant solubility, distinguishing LAG conditions from slurry reactions (1 µL/mg < η < 2 µL/mg) in which low solubility does hinder reactivity. (Friščić et al., ACS Cent. Sci. 2017, 3(1), 13-19)

As used herein, the terms "minimum or limited amounts of a liquid" or "minimum /small amount of solvent" refers to an eta value (*η*) up to 1, compared to the same reaction without mechanochemical conditions".

The compounds involved in the Invention are the ones of formula (I), their enantiomers, in particular under pure form, or a mixture of said enantiomers, in particular a racemic mixture.

The formula (I) encompasses compounds of formula (III): 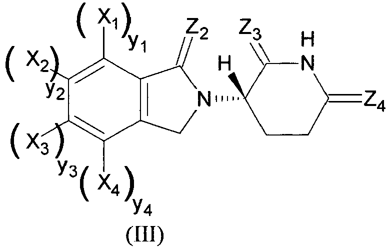
wherein Z₂, Z₃, Z₄, X₁, X₂, X₃, X4, y₁, y₂, y₃ and y₄ have the meaning defined above and compounds of formula (IV): 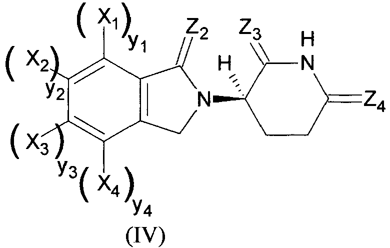
wherein Z₂, Z₃, Z₄, X₁, X₂, X₃, X4, y₁, y₂, y₃ and y₄ have the meaning defined above,
that are part of the invention.

The expression "(C₁-C₅)alkyl" group means an alkyl group having from 1 to 5 carbon atoms.

The expression "(C₁-C₅)alkoxy" group means an alkyl group from 1 to 5 carbon atoms, which is singularly bonded to oxygen.

The expression "(H or C₁-C₁₀)acyl group" means a C₁ - C₁₀ 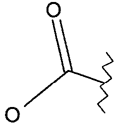 group.

The expression" halogen atom" means an atom chosen among: fluorine, chlorine, bromine or iodide.

The expression "pharmaceutically acceptable salt" means all pharmaceutically or physiologically acceptable salt forms of the compounds of compound of formula (I) which may be formed, by protonation of a nitrogen, with an inorganic or organic acid. Examples acid addition salts comprise, mineral acid salts such as hydrochloride, hydrobromide, hydroiodide, sulfate salts (such as, e.g., sulfate or hydrogensulfate salts), phosphate salts (such as hexafluorophosphate, hydrogenphosphate, or dihydrogenphosphate salts), carbonate salts, hydrogenocarbonate salts, borate salts, organic acid salts such as oxalate, fumarate, tartrate, malate, citrate, succinate, adipate, gluconate, glycolate, nicotinate, benzoate, salicylate, ascorbate salts; sulfonate salts such as methanesulfonate (mesylate), ethanesulfonate (esylate), 2-hydroxyethanesulfonate (isethionate), p-toluenesulfonate (tosylate), or camphorsulfonate salts; glycerophosphate salts; and acidic amino acid salts such as aspartate or glutamate salts. Preferred pharmaceutically/physiologically acceptable salt of compound of formula (I) include hydrochloric acid, hydrobromic acid, methane sulfonic acid, ethane sulfonic acid, benzene sulfonic acid and p-toluenesulfonic acid.

The starting product in the preparation of a compound of formula (I) is 3-aminopiperidine-2,6-dione hydrochloride (CAS number: 24666-56-6) is a salt of a cyclic dione compound containing two nitrogens. It consists of a six-membered ring, in presence with a hydrochloride salt.

"wherein at least one step is mechanochemical" refers to the fact that in the process of preparation of compound of formula (I), the minimum number of step that is performed thanks to mechanical activation is 1.

According to another embodiment, the invention consists in the use of a mechanical force to generate a mechanochemical reaction in the implementation of a process of preparation, of a compound of formula (I) or a pharmaceutically acceptable salt thereof, said process comprising 2 steps, using 3-aminopiperidine-2,6-dione as starting material, wherein at least one step is mechanochemical.

According to a particular embodiment of the use of the invention, the process of preparation comprises a cycloamidation step (which is mechanochemical) and a step of mechanochemical reduction.

According to a particular embodiment, the present invention concerns the preparation of the compound of formula (I), wherein y₁ = y₂ = y₃ = 0, y₄ = 1, Z₂, Z₃ and Z₄ are oxygen atoms and X₄ is an amino group, and corresponding to the following formula (Ia):

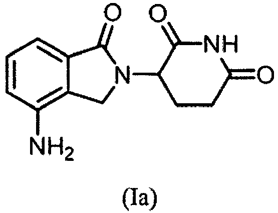

The compound of formula (Ia) corresponds to lenalidomide (CAS number : 191732-72-6).

According to a particular embodiment of the invention, said process is carried out in batch or in flow. The expression "batch" means a type of process carried out in a closed system, where the totality chemical reactants are added at the beginning, and the process proceeds without further input or removal of materials until the end of the process.

The expression "flow" means is a type of process carried out in a continuously flowing system, where reactants are introduced and products are removed simultaneously.

According to a particular embodiment of the invention, the mechanical forces are generated by a method chosen among: ball-milling (BM), in particular vibrating ball-milling (VBM) or planetary ball-milling (PBM), resonant acoustic mixing (RAM) or screw extrusion (single, twin or multi).

Examples of the reaction vessel and the balls as an agitation medium used in the milling devices include ones formed of such materials as stainless steel, agate, alumina, tungsten carbide, chrome steel, zirconia oxide, silicon nitride and the like. Among these materials, zirconia oxide, is preferred. The size of the vessel used in the planetary ball mill is not particularly limited and may be approximately 1 cm³ to 1000 L. The size of the balls is also not particularly limited and may be approximately from 2 to 50 mm in diameter. Particularly preferred specific examples of the vibrating and/or planetary ball mill include stainless steel and zirconia oxide as reaction vessel material.

Resonant acoustic mixing (RAM) involves applying acoustic energy at a frequency of 60Hz while performing a vibrating oscillating movement reciprocating up and down motions on the contents of the container. This movement may be applied with a relatively high (up to 100 g) acceleration and a relatively low amplitude. The frequency of the vibration is typically around 60 Hz. The container can be cylindrical shaped with a circumferential side wall and disc-shaped top and bottom walls. The force is applied along the longitudinal axis of the container.

In resonant acoustic mixing, acoustic energy is delivered to the components to be mixed. An oscillating mechanical driver creates motion in a mechanical system comprising engineered plates, eccentric weights and springs. This energy is then acoustically transferred to the material to be mixed. The underlying technology principle is that the system operates at resonance. In this mode, there is a nearly complete exchange of energy between the mass elements and the elements in the mechanical system.

The resonant acoustic mixing provides a highly efficient way of transferring mechanical energy directly into the starting materials. The resonant frequency can be from about 15 Hertz to about 2000 Hertz, or from about 20 Hertz to about 1800 Hertz, or from about 20 Hertz to about 1700 Hertz. The g force applied by the acoustic mixer to the reactant composition can be from about 2 g force to about 100 g force.

Resonant acoustic mixers are available from Resodyn^{™} Acoustic Mixers and can be chosen from LabRAM I and LabRAM II, RAMS, RAM55, OmniRAM and like.

Screw extrusion may be performed at various speeds (S_{S}), screw temperatures (S_{T}) and residence times (T_{R}), as described herein. A single pass through the extruder may be sufficient to form the product of a mechanochemical reaction. Alternatively, when step a) is conducted in a twin-screw extruder, step a) may comprise collecting the product emerging from the twin screw extruder and subjecting it to one or more additional passes through the simple or twin-screw extruder.

Multi-screw extruders for use herein are not particularly limited. Any multi-screw extruder comprising a reaction chamber commonly known in the art may be used in the context of the present disclosure. Suitable multi-screw extruders for use herein will be easily identified by those skilled in the art, in the light of the present description.

In a particular aspect of the present application, the screw extruder for use herein is selected from the group of Single Screw Extruders (Helibar Transfer Mix, Co-Kneader), Twin Screw Extruders: 1. Intermeshing Screws: Co-rotating Parallel Screws, Counter-rotating Parallel Screws), 2: Non-intermeshing Screws (Co-rotating Parallel Screws, Co-rotating Conical Screws, Counter-rotating Screws), 3: Multiple Screw Extruders (Rotating Center Shaft: Planetary Roller Extruder), Static Center Shaft (Ring Extruder, Multiple Screw Extruder).

The main extruder parameters to consider are:
- feed rate of chemical reactants forming the starting material mixture: from 0.1 g/min to 10 g/min for an extruder that is adapted to a laboratory (as a matter of example and in a non-limiting manner, with 9 mm, (length/diameter ratio) 25:1 TSE; from 0.4 to 1.5 g/min) and for larger extruders in the industry, from 1 kg/min to 50 kg/min)
- Number of the liquid inlet ports and their position (as a matter of example and in a non-limiting manner with ZE9 Three-Tec extruder (LD 25:1) of Three Tec company, 2 liquid ports are available: one close to the beginning of the barrel and one close to the end) used for the addition of liquid additive (the flow rate of liquid additive depends on the eta value and the feed rate of solid chemical reactants)
- Presence or not of a die and the type of die (The dies are defined and manufactured according to process requirements. Die diameters are possible from 0.1 mm. The empty volume in the die is negligible, as the screws extend into the die until just before the opening. Due to the simple design and low mass, separate die heating can be dispensed with in most cases. If heating of the die is nevertheless necessary, the same heating elements as for the barrel can be used for this purpose. The following die geometries are possible: Single and double dies, Sieve dies, Film dies (fixed or adjustable), Hose dies, Cooling dies for meat substitutes).
- Screw speed (from 20 to 1000 RPM) (as a matter of example and in a non-limiting manner Hybrid ZE9/ZE12 Three-Tec extruder of Three Tec company can rotate from 20 to 200 RPM).
- Barrel temperature (as a matter of example and in a non-limiting manner, the hybrid ZE 9/12 Three-Tec TSE extruder of Three Tec company is able to heat from 25°C to 400°C, more preferably from 25°C to 230 °C.
- Screw profile: Modular allow infinite configuration of screw profiles by replacing some screw elements by others. Screw elements can be: conveying element (reverse or not), mixing elements (reverse or not) and kneading elements.

According to a particular embodiment of the invention, at least one step is mechanochemical.

According to a particular embodiment of the use of the invention, wherein at least two steps are mechanochemical.

According to a particular embodiment of the use of the invention, wherein said process is carried out in flow and wherein at least two steps are mechanochemical.

According to a particular embodiment of the invention, said process of preparation provides at least 3.10³ kg.m⁻³ of said reactor.day⁻¹, in particular from 3.10³ kg.m⁻³ of said reactor.day⁻¹ to 68 000 kg.m⁻³ of said reactor.day⁻¹, of said compound of formula (I) or of said pharmaceutically acceptable salt thereof.

By range of "from 3.10³ kg.m⁻³ of said reactor.day⁻¹ to 68 000 kg.m⁻³" it means: from 3 000 to 4 000, from 4 000 to 8 000, from 8 000 to 12 000, from 12 000 to 16 000, from 16 000 to 20 000, from 20 000 to 24 000, from 24 000 to 28 000, from 28 000 to 32 000, from 32 000 to 36 000, from 36 00 to 40 000, from 40 000 to 44 000, from 44 000 to 48 000, from 48 000 to 52 000, from 52 000 to 56 000, from 56 000 to 60 000, from 60 000 to 64 000 and from 64 000 to 68 000 kg.m⁻³ of said reactor.day⁻¹.

According to a preferred embodiment of the invention, the process comprises a cycloamidation step of 3-aminopiperidine-2,6-dione hydrochloride with methyl 2-(bromomethyl)-3-nitrobenzoate, a base chosen among tripotassium phosphate and potassium carbonate, and an additive, into 3-(4-nitro-1-oxoisoindolin-2-yl) piperidine-2,6-dione, said cycloamidation step is mechanochemical,
and/or
comprises a step of mechanochemical reduction of the above mentioned 3-(4-nitro-1-oxoisoindolin-2-yl) piperidine-2,6-dione into a compound of formula (Ia) 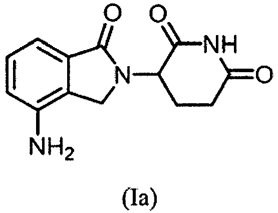
with at least one additive and sodium dithionite.

The cycloamidation step in this embodiment consists in the formation of an amide function and a cyclization, represented in the following scheme: 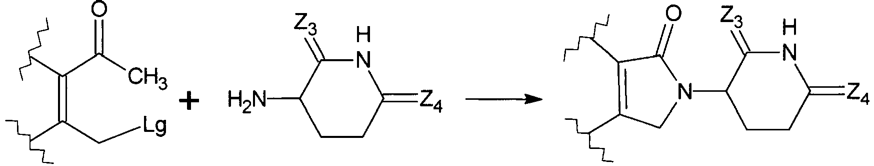 wherein Lg represents a nucleofuge (or a chemical leaving group), in particular a bromine atom, and Z₃ and Z₄ have the meaning defined above. In a non-limiting manner, exemplary nucleofuges are halogen, hydroxysuccinimide, paratoluenesulfonate and alkylsulfonate. The cycloamidation proceeds by a nucleophilic substitution and a leaving of the nucleofuge.

Methyl 2-(bromomethyl)-3-nitrobenzoate corresponds to 98475-07-1 as CAS registry number.

An additional advantage of the invention concerns the cycloamidation reaction in solution that occurs in about 15 hours. The mechanochemical reaction lasts about 2h using BM and RAM,-while by twin screw extrusion (TSE), the amidation method lasts about 60 min.

The step of mechanochemical reduction in this embodiment consists in the reduction of at least one nitro group into an amino group, represented in a non-limiting manner by the following scheme: 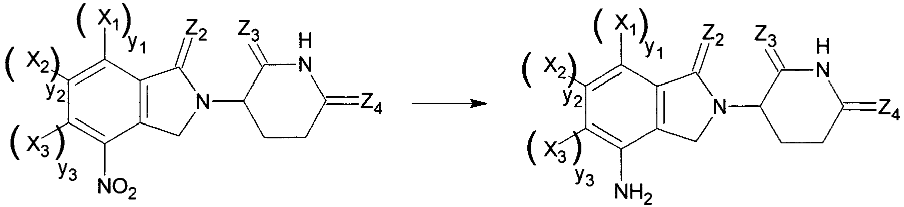 wherein:
- Z₂, Z₃ and Z₄ having the meaning defined above,
- X₁, X₂ and X₃, identical or different, are chosen among: (C₁-C₅)alkyl group, (C₁-C₅)alkoxy group, halogen atom, trifluoromethyl group, hydroxy group, (H or C₁-C₁₀)acyl group, a 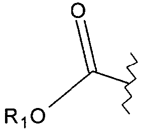 group, or a 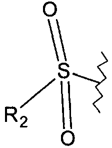 group
   wherein R₁ is chosen among: hydrogen atom, (C₁-C₅)alkyl group, or -NR₁ₐR_{1b} group, R₁ₐ and R_{1b}, identical or different, are chosen among: hydrogen atom or (C₁-C₅)alkyl group,
   and R₂ is chosen among: hydroxy group, (C₁-C₅)alkoxy group, -NR₂ₐR_{2b},
   R₂ₐ and R_{2b}, identical or different, are chosen among: hydrogen atom or (C₁-C₅)alkyl group
- y₁, y₂, y₃ and y₈, identical or different, are equal to 0 or 1, and provided that y₁ + y₂ + y₃ is 1.

Another object of the present invention is a mechanochemical process of preparation of compound of formula (I).

In a particular embodiment, the invention is a mechanochemical process of preparation of a compound of formula (I), 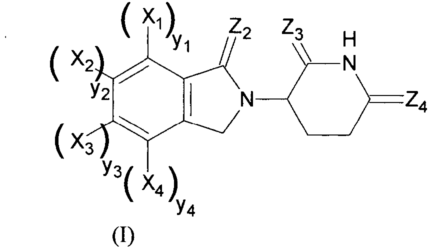 wherein:
- Z₂, Z₃ and Z₄, identical or different, are chosen among: oxygen or sulfur atom,
- X₁, X₂, X₃ and X₄, identical or different, are chosen among: (C₁-C₅)alkyl group, (C₁-C₅)alkoxy group, amino group, halogen atom, trifluoromethyl group, hydroxy group, (H or C₁-C₁₀)acyl group, a 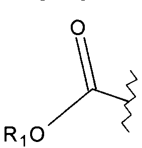 group, or a 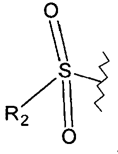 group
   wherein R₁ is chosen among: hydrogen atom, (C₁-C₅)alkyl group, or -NR₁ₐR_{1b} group,
   R₁ₐ and R_{1b}, identical or different, are chosen among: hydrogen atom or (C₁-C₅)alkyl group,
   and R₂ is chosen among: hydroxy group, (C₁-C₅)alkoxy group, -NR₂ₐR_{2b},
   R₂ₐ and R_{2b}, identical or different, are chosen among: hydrogen atom or (C₁-C₅)alkyl group
- y₁, y₂, y₃ and y₄, identical or different, are equal to 0 or 1, and provided that y₁ + y₂ + y₃ + y₄ is 1 or 2,
- and provided that at least one X₁, X₂, X₃ and X₄ is an amino group,
said process comprising:
a) a cycloamidation step, using 3-aminopiperidine-2,6-dione hydrochloride as starting material, and a carboxylic function bearer, said carboxylic function bearer being in particular methyl 2-(bromomethyl)-3-nitrobenzoate,
   a base in particular chosen among tripotassium phosphate and potassium carbonate, and optionally at least one additive, in particular said at least one additive being sodium chloride, said process using mechanochemical forces, and
   being carried out under neat grinding, or in solid-assisted grinding (SAG) conditions, or in liquid-assisted grinding (LAG) conditions, or in LAG conditions with a solid additive,
   to obtain a crude product containing a compound of formula (II): 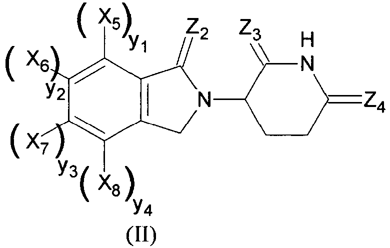
   wherein:
      - Z₂, Z₃ and Z₄ having the meaning defined above,
      - X₅, X₆, X₇ and X₈, identical or different, are chosen among: (C₁-C₅)alkyl group, (C₁-C₅)alkoxy group, nitro group, halogen atom, trifluoromethyl group, hydroxy group, (H or C₁-C₁₀)acyl group, a 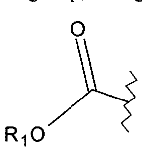 group, or a 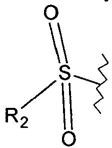 group
         wherein R₁ is chosen among: hydrogen atom, (C₁-C₅)alkyl group, or -NR₁ₐR_{1b} group, R₁ₐ and R_{1b}, identical or different, are chosen among: hydrogen atom or (C₁-C₅)alkyl group,
         and R₂ is chosen among: hydroxy group, (C₁-C₅)alkoxy group, -NR₂ₐR_{2b},
         R₂ₐ and R_{2b}, identical or different, are chosen among: hydrogen atom or (C₁-C₅)alkyl group
      - y₅, y₆, y₇ and y₈, identical or different, are equal to 0 or 1, and provided that y₅ + y₆ + y₇ + y₈ is 1 or 2,
      - and provided that at least one X₅, X₆, X₇ and X₈ is a nitro group,
         and,
b) a step of mechanochemical reduction of the above-mentioned compound of formula (II), with at least one additive, using mechanochemical forces, in liquid-assisted grinding (LAG)
   conditions with sodium dithionite, to obtain a crude product containing said compound of formula (I).

The expression "carboxylic function bearer" refers to a compound a formula (VII): 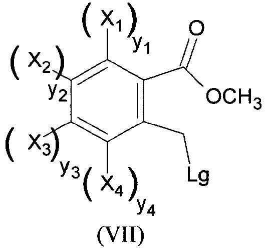 wherein X₁, X₂, X₃, X₄, y₁, y₂, y₃, y₄ and Lg have the meaning defined above.

Mechanochemistry reactions can require the addition of a solid additive to improve the rheology for efficient reactivity. It has been shown that different textures of reaction mixtures can lead to different reaction kinetics, the reaction mixture can in some cases become sticky and resembles a paste, this prevents efficient mass and energy transfer. To take this into account, it is common to use a solid additive such as silica, alumina, talc, celite or inorganic salts (e.g., NaCl or NaBr) and other chemically inert salts as a milling agent/auxiliary or adsorbent.

Solid additives have the function to improve the rheology of the reaction during the mechanochemical process.

Liquid additive can also be selected in the group of any solvent, polar or apolar (hexane, ethyl acetate, acetone, acetonitrile, dimethyl sulfoxide, dimethyl isosorbide, C₁-C₁₀ alcohol, isopropanol), bio-sourced or not, low melting polymers, deep eutectic solvents (DES), including ionic liquids of any type, such as and in a non-limiting manner, DES choline chloride/glycerol (1 : 2, mol/mol), also known as glyceline or choline chloride/ethylene glycol (1 : 2, mol/mol).

The expression "neat grinding" corresponds to the mechanical force that takes place in absence of solid or liquid additive.

The expression "solid-assisted grinding (SAG) conditions" refers to the mechanical force that takes place in presence of solid additive that does not participate in the reaction mechanism and in absence of a liquid additive.

The expression "a crude product containing" means that during one of the step a) or b), a crude product containing various traces of unwanted reagents is obtained, and this crude product must go through a purification to give the desired product.

The expression "LAG conditions with a solid additive" refers to the mechanical force that takes place in the presence of a liquid additive that improves the mixing of the reaction and may/may not participate in the reaction mechanism in a catalytic way and where the rheological properties of the mixture are improved by the presence of a solid additive.

In the case of BM, using NaCl as additive avoids the presence of impurities and improves the rheology of the reaction mixture. In the case of RAM activated reactions, the solid additive is not necessary and only liquid additives in the process is needed. In both cases (BM and RAM), the work-up procedures are simplified, because the product can be easily recovered avoiding purifications steps, otherwise needed when impurities are formed. This has also the advantage to improve the environmental footprint of the entire process, also reducing the amount of waste generated.

According to a particular embodiment of the process of the invention, wherein the step a) is carried out with at least one additive.

According to a particular embodiment, in the process of the invention, the step a) is carried out with at least one additive, and:
- when said at least one additive is solid under mechanical forces, the step a) is carried out in solid-assisted grinding (SAG) conditions,
   or
- when said at least one additive is liquid under mechanical forces, the step a) is carried out in liquid-assisted grinding (LAG) conditions,
   or
- when said at least one additive comprises a solid additive and a liquid additive under mechanical forces, the step a) is carried out in LAG conditions with a solid additive.

According to a particular embodiment in the process of the invention, the step a) is carried out with a liquid additive in liquid-assisted grinding (LAG) conditions.

According to a particular embodiment of the process of the invention, in the step a) the solid additive is sodium chloride and the ratio of carboxylic function bearer/additive is from 0.01 to 10, in particular 0.10.

By range of "from 0.01 to 10" it means: from 0.01 to 0.10, from 0.10 to 1 and from 1 to 10".

According to a particular embodiment of the process of the invention, in step a) and/or in step b), the mechanical forces are generated by a method chosen among: ball-milling (BM), in particular vibrating ball-milling (VBM), planetary ball-milling (PBM), resonant acoustic mixing (RAM) or screw extrusion (single, twin or multi).

The advantages of this embodiment are:
- for step a) and step b) wherein the mechanical forces are generated by BM:
   1) to reduce the reaction times to 2h or 3h (examples 1 and 2 respectively), compared to the manufacturing process in solution, which requires 15 h:
   2) to avoid the use of (toxic) organic bases (e.g., triethylamine), which are herein replaced by the inorganic (non-toxic) base K₂CO₃, leading to an improved eco footprint of the process;
   3) to avoid the heating at 80°C, the reaction occurring at room temperature, making the process less-energy demanding;
   4) to avoid the use of harmful (and high boiling temperature) solvents such as DMF, also responsible for the formation of toxic impurities such as nitrosamines, usually formed during the manufacturing process in solution, especially upon heating. Nitrosamines are harmful and toxic compounds posing risks for the patient health.
- for step a) wherein the mechanical forces are generated by RAM:
   1) to reduce the reaction times to 2h or 3h compared to the manufacturing process in solution, which requires 15 h:
   2) to avoid the use of (toxic) organic bases (e.g., triethylamine), which are herein replaced by the inorganic (non-toxic) bases (e.g., K₂CO₃, K₃PO₄) leading to an improved eco footprint of the process;
   3) to avoid the heating at 80°C, the reaction occurring at room temperature, making the process less-energy demanding;
   4) to avoid the use of harmful (and high boiling temperature) solvents such as DMF, also responsible for the formation of toxic impurities such as nitrosamines, usually formed during the manufacturing process in solution, especially upon heating. Nitrosamines are harmful and toxic compounds posing risks for the patient health.
- for step b) wherein the mechanical forces are generated by RAM:
   1) reduction of the reaction times to 1h compared to the manufacturing process in solution, which requires 6.5 h:
   2) circumvent the use of precious, toxic and costly raw materials such as metal-based catalysts, for which shortage is also an issue (e.g., palladium catalyst), also making the recovery of the final product cumbersome (for the catalyst separation) and generating toxic waste. The reduction by RAM is 'metal-free', make use cheap reagent (e.g. Na₂S₂O₄), generate not-harmful waste, leading to an improved eco-footprint of the process;
   3) avoid the heating at 50-60°C, the reaction occurring at room temperature, making the process less-energy demanding;
   4) avoid the use of harmful (and high boiling temperature) solvents such as 1,4-dioxane.
- for step a) and step b) wherein the mechanical forces are generated by TSE:
   1) to avoid the use of (toxic) organic bases (e.g., triethylamine), which are herein replaced by the inorganic (non-toxic) base (*e.g.,* K₂CO₃) leading to an improved eco footprint of the process;
   2) to avoid the heating at 80°C, the reaction occurring at room temperature, making the process less-energy demanding;
   3) to avoid the use of harmful (and high boiling temperature) solvents such as DMF, also responsible for the formation of toxic impurities such as nitrosamines, usually formed during the manufacturing process in solution, especially upon heating. Nitrosamines are harmful and toxic compounds posing risks for the patient health.

According to a particular embodiment of the process of the invention, when the mechanical forces are generated by RAM, said mechanochemical process is carried out in a vessel wherein a m x g force is applied, m being a positive number.

In this embodiment, the vessel is made of a material chosen, as a matter of example and in a non-limiting way among: glass, pyrex^{®}, polypropylene or stainless steel, and has a volume comprised from 2 mL to 2L.

According to a particular embodiment of the process of the invention, the g force is from about 2 g to about 100 *g*.

According to an advantageous embodiment of the process of the invention, the mechanical forces are generated by ball-milling (BM).

**FOR STEP a (Examples 1 and 2 hereafter):** The reaction by ball-milling brings the following improvements compared to manufacturing process in solution:
1) to reduce the reaction times to 2h or 3h (examples 1 and 2 respectively), compared to the manufacturing process in solution, which requires 15 h:
2) to avoid the use of (toxic) organic bases (e.g., triethylamine), which are herein replaced by the inorganic (non-toxic) base K₂CO₃, leading to an improved eco footprint of the process;
3) to avoid the heating at 80°C, the reaction occurring at room temperature, making the process less-energy demanding;
4) to avoid the use of harmful (and high boiling temperature) solvents such as DMF, also responsible for the formation of toxic impurities such as nitrosamines, usually formed during the manufacturing process in solution, especially upon heating. Nitrosamines are harmful and toxic compounds posing risks for the patient health.

According to a particular embodiment, the process of the invention enables to prepare a compound of formula (V): 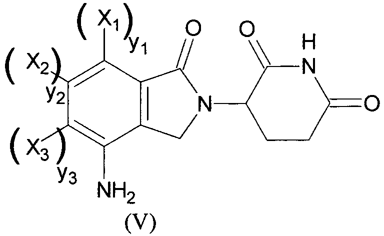
wherein X₁, X₂, X₃, y₁, y₂ and y₃ have the meaning defined above,
   in particular of formula (Ia) 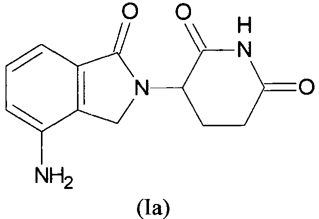
said process comprising:
   a) a cycloamidation step, wherein 3-aminopiperidine-2,6-dione hydrochloride is used as starting material
      said carboxylic function bearer is methyl 2-(bromomethyl)-3-nitrobenzoate,
      and said base is in particular K₂CO₃
      and said at least one additive is, in particular sodium chloride, dimethyl sulfoxide or dimethyl isosorbide, or a mixture of thereof,
      and said mechanical forces are generated by BM and are carried out in SAG conditions, or in LAG conditions or in LAG conditions with a solid additive,
      to obtain a crude product containing a compound of formula (VI), 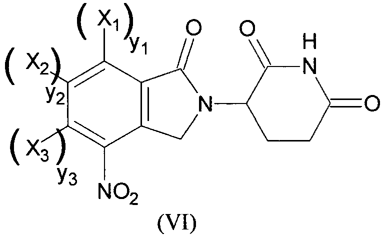 wherein X₁, X₂, X₃, y₁, y₂ and y₃ have the meaning defined above,
         - wherein when the step a) is carried out in SAG conditions, the SAG conditions are defined by the ratio of carboxylic function bearer/additive that is from 0.01 to 10, in particular 0.10,
         - wherein when the step a) is carried out in LAG conditions, the LAG conditions are defined by the parameter η that is greater than 0 µL/mg and less than or equal to 1 µL/mg, more preferably is comprised from about 0.05 µL/mg to 0.35 µL/mg, more preferably 0.2 µL/mg,
         - wherein when the step a) is carried out in LAG conditions with a solid additive, the in LAG conditions with a solid additive are defined :
            ➢ by the ratio of carboxylic function bearer/additive, that is from 0.01 to 10, in particular 0.10, and
            ➢ by the parameter η that is greater than 0 µL/mg and less than or equal to 1 µL/mg, more preferably is comprised from about 0.05 µL/mg to 0.35 µL/mg, more preferably 0.2 µL/mg,
      and
   b) a step of mechanochemical reduction of the above-mentioned compound of formula (VI), said mechanical forces being generated by BM, with said at least one additive, to obtain a crude product containing said compound of formula (V),
      wherein in the step b), the LAG conditions are defined by the parameter η that is greater than 0 µL/mg and less than or equal to 1 µL/mg, preferably is comprised from about 0.25 µL/mg to 0.75 µL/mg, more preferably 0.5 µL/mg.

According to a particular embodiment of the process of the invention, when the step a) is in LAG or in LAG conditions with a solid additive, the said at least one additive comprises at least one liquid additive which is dimethyl sulfoxide.

According to a particular embodiment of the process of the invention, when the step a) is in LAG or in LAG conditions with a solid additive, the said at least one additive comprises at least one liquid additive which is dimethyl isosorbide (DMI).

According to a particular embodiment of the process of the invention, in step a), the ratio of methyl 2-(bromomethyl)-3-nitrobenzoate/sodium chloride is 0.10.

According to a particular embodiment of the process of the invention, in step b), the said at least one additive is liquid and is a mixture of water and an alcohol, in particular ethanol.

According to a particular embodiment of the process of the invention, the compound of formula (VI) is recovered from the crude product containing it, by a washing with water, a filtration and a removal of said water.

According to a particular embodiment of the process of the invention, the compound of formula (V) is recovered from the crude product containing it, by a washing with boiling solvent, in particular acetone, by a filtration and by an evaporation of said solvent.

As a matter of example and in a non-limiting manner, the step a) can be carried out with a mechanochemical device operating at 20-22°C, with methyl 2-(bromomethyl)-3-nitrobenzoate as the carboxylic acid bearer, 3-aminopiperidine-2,6-dione hydrochloride as the starting material, K₂CO₃ as the base, dimethyl isosorbide as liquid additive and NaCl as solid additive in LAG conditions with a solid additive.

As a matter of example and in a non-limiting manner, the step a) can be carried out with a mechanochemical device operating at 20-22°C, with methyl 2-(bromomethyl)-3-nitrobenzoate as the carboxylic acid bearer, 3-aminopiperidine-2,6-dione hydrochloride as the starting material, K₃PO₄ as the base, dimethyl sulfoxide as liquid additive and NaCl as solid additive in LAG conditions with a solid additive.

According to an advantageous embodiment of the process of the invention, the mechanical forces are generated by resonant acoustic mixing (RAM).

**FOR STEP a (Examples 4-8 hereafter):** The reaction by resonant acoustic mixing (RAM) brings the following improvements compared to manufacturing process in solution:
1) to reduce the reaction times to 2h or 3h compared to the manufacturing process in solution, which requires 15 h:
2) to avoid the use of (toxic) organic bases (e.g., triethylamine), which are herein replaced by the inorganic (non-toxic) bases (e.g., K₂CO₃, K₃PO₄) leading to an improved eco footprint of the process;
3) to avoid the heating at 80°C, the reaction occurring at room temperature, making the process less-energy demanding;
4) to avoid the use of harmful (and high boiling temperature) solvents such as DMF, also responsible for the formation of toxic impurities such as nitrosamines, usually formed during the manufacturing process in solution, especially upon heating. Nitrosamines are harmful and toxic compounds posing risks for the patient health.

**FOR STEP b (Example 9 hereafter):** The reduction reaction by resonant acoustic mixing (RAM) brings the following improvements compared to manufacturing process in solution:
1) reduction of the reaction times to 1h compared to the manufacturing process in solution, which requires 6.5 h:
2) circumvent the use of precious, toxic and costly raw materials such as metal-based catalysts, for which shortage is also an issue (e.g., palladium catalyst), also making the recovery of the final product cumbersome (for the catalyst separation) and generating toxic waste. The reduction by RAM is 'metal-free', make use cheap reagent (e.g. Na₂S₂O₄), generate not-harmful waste, leading to an improved eco-footprint of the process;
3) avoid the heating at 50-60°C, the reaction occurring at room temperature, making the process less-energy demanding;
4) avoid the use of harmful (and high boiling temperature) solvents such as 1,4-dioxane.

According to a particular embodiment, the process of the invention concerns the preparation of a compound of formula (V): 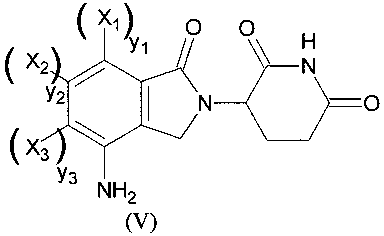
wherein X₁, X₂, X₃, y₁, y₂ and y₃ have the meaning defined above,
   in particular of formula (Ia) 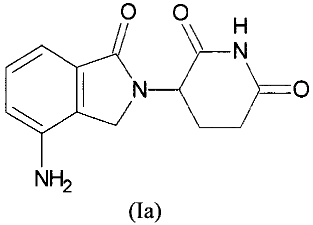
said process comprising:
   a) a cycloamidation step, wherein 3-aminopiperidine-2,6-dione hydrochloride is used as starting material
      said carboxylic function bearer is methyl 2-(bromomethyl)-3-nitrobenzoate and said base is in particular K₃PO₄ or K₂CO₃,
      and said at least one additive is used,
      and said mechanical forces are generated by RAM and are carried out in LAG conditions or in LAG conditions with a solid additive, in particular sodium chloride,
      to obtain a crude product containing a compound of formula (VI), 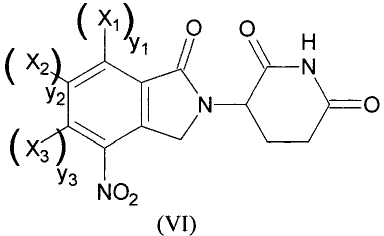 wherein X₁, X₂, X₃, y₁, y₂ and y₃ have the meaning defined above,
         - wherein when the step a) is carried out in LAG conditions, the LAG conditions are defined by the parameter η that is greater than 0 µL/mg and less than or equal to 1 µL/mg, preferably is comprised from about 0.25 µL/mg to 0.75 µL/mg, more preferably 0.25 µL/mg,
            or
         - wherein when the step a) is carried out in LAG conditions with a solid additive, the LAG conditions with a solid additive are defined:
            ➢ by the ratio of carboxylic function bearer/additive, that is from 0.01 to 10, in particular 0.10, and
            ➢ by the parameter η that is greater than 0 µL/mg and less than or equal to 1 µL/mg, more preferably is comprised from about 0.05 µL/mg to 1 µL/mg, more preferably 0.2 µL/mg,
               and
   b) a step of mechanochemical reduction of the above-mentioned compound of formula (VI), said mechanical forces being generated by RAM, with said at least one additive, to obtain a crude product containing said compound of formula (V),
      wherein in the step b), the LAG conditions are defined by the parameter η that is greater than 0 µL/mg and less than or equal to 1 µL/mg, preferably is comprised from about 0.60 µL/mg to 0.90 µL/mg, more preferably 0.75 µL/mg.

According to a particular embodiment of the process of the invention, in the step a), the said at least one additive is liquid and is dimethyl sulfoxide.

According to a particular embodiment of the process of the invention, when the step a) is in LAG or in LAG conditions with a solid additive, the said at least one additive comprises at least one liquid additive which is dimethyl sulfoxide.

According to a particular embodiment of the process of the invention, when the step a) is in LAG or in LAG conditions with a solid additive, the said at least one additive comprises at least one liquid additive which is dimethyl isosorbide (DMI).

According to a particular embodiment of the process of the invention, said base is K₃PO₄,
wherein the step a) is in LAG conditions, said at least one additive comprises at least one liquid additive which is dimethyl isosorbide (DMI), and the LAG conditions are defined by the parameter η that is greater than 0 µL/mg and less than or equal to 1 µL/mg, preferably is comprised from about 0.50 µL/mg to 1 µL/mg, more preferably is 0.75 µL/mg.

According to a particular embodiment of the process of the invention, said base is K₂CO₃,
the step a) is in LAG conditions, said at least one additive comprises at least one liquid additive which is dimethyl isosorbide (DMI), and the LAG conditions are defined by the parameter η that is greater than 0 µL/mg and less than or equal to 1 µL/mg, preferably is comprised from about 0.29 µL/mg to 0.89 µL/mg, more preferably is 0.59 µL/mg.

According to a particular embodiment of the process of the invention, in step a), the ratio of methyl 2-(bromomethyl)-3-nitrobenzoate/sodium chloride is 0.10.

According to a particular embodiment of the process of the invention, in step b), the said at least one additive is liquid and is a mixture of water and an alcohol, in particular ethanol.

According to a particular embodiment of the process of the invention, in step a), the g force is comprised from 2 g to 100 g, in particular 90 g.

According to a particular embodiment of the process of the invention, in step b), the g force is comprised from 2 g to 100 g, in particular 90 g.

According to a particular embodiment of the process of the invention, the compound of formula (VI) is recovered from the crude product containing it, by a washing with water, a filtration and a removal of said water.

According to a particular embodiment of the process of the invention, the compound of formula (V) is recovered from the crude product containing it, by a first filtration to obtain a filtrate, placing said filtrate in contact with an aqueous solution of hydrochloric acid, a removal of said solution of hydrochloric acid, placing said filtrate in contact with an aqueous solution of sodium hydrogencarbonate, a second filtration to obtain a second filtrate, and a removal of said aqueous solution of sodium hydrogencarbonate from the second filtrate.

As a matter of example and in a non-limiting manner, the step a) can be carried out with a mechanochemical device operating at 20-22°C, with methyl 2-(bromomethyl)-3-nitrobenzoate, 3-aminopiperidine-2,6-dione hydrochloride, K₃PO₄, dimethyl isosorbide as liquid additive in a 10 mL glass vial in LAG conditions.

As a matter of example and in a non-limiting manner, the step a) can be carried out with a mechanochemical device operating at 20-22°C, with methyl 2-(bromomethyl)-3-nitrobenzoate as the carboxylic acid bearer, 3-aminopiperidine-2,6-dione hydrochloride as the starting material, K₃PO₄ as the base, and dimethyl sulfoxide as liquid additive in LAG conditions.

As a matter of example and in a non-limiting manner, the step a) can be carried out with a mechanochemical device operating at 20-22°C, with methyl 2-(bromomethyl)-3-nitrobenzoate as the carboxylic acid bearer, 3-aminopiperidine-2,6-dione hydrochloride as the starting material, K₂CO₃ as the base and dimethyl isosorbide as liquid additive in a 15ml PTFE jar in LAG conditions.

As a matter of example and in a non-limiting manner, the step a) can be carried out with a mechanochemical device operating at 20-22°C, with methyl 2-(bromomethyl)-3-nitrobenzoate as the carboxylic acid bearer, 3-aminopiperidine-2,6-dione hydrochloride as the starting material, K₂CO₃ as the base, and dimethyl isosorbide as liquid additive in LAG conditions with sodium chloride as solid additive.

According to an advantageous embodiment of the process of the invention, the mechanical forces are generated by twin screw extrusion (TSE).

**FOR STEP a (Example 10 hereafter):** The reaction by TSE brings the following improvements compared to manufacturing process in solution:
1) to avoid the use of (toxic) organic bases (e.g., triethylamine), which are herein replaced by the inorganic (non-toxic) base (e.g., K₂CO₃) leading to an improved eco footprint of the process;
2) to avoid the heating at 80°C, the reaction occurring at room temperature, making the process less-energy demanding;
3) to avoid the use of harmful (and high boiling temperature) solvents such as DMF, also responsible for the formation of toxic impurities such as nitrosamines, usually formed during the manufacturing process in solution, especially upon heating. Nitrosamines are harmful and toxic compounds posing risks for the patient health.

According to a particular embodiment, the process of the invention concerns the preparation of a compound of formula (V): 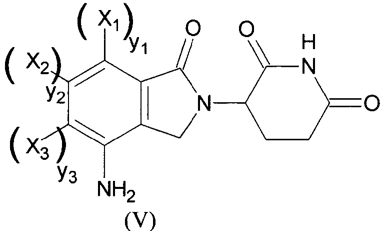
wherein X₁, X₂, X₃, y₁, y₂ and y₃ have the meaning defined above,
   in particular of formula (Ia) 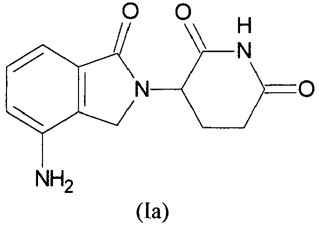
said process comprising:
   a) a cycloamidation step, wherein 3-aminopiperidine-2,6-dione hydrochloride is used as starting material,
      said carboxylic function bearer is methyl 2-(bromomethyl)-3-nitrobenzoate,
      and said base is in particular K₂CO₃,
      and said at least one additive is in particular sodium chloride, dimethyl sulfoxide, or dimethyl isosorbide, or a mixture of thereof,
      and said mechanical forces are generated by twin screw extrusion, and are carried out in SAG, or LAG or in LAG conditions with a solid additive,
      to obtain a crude product containing a compound of formula (VI), 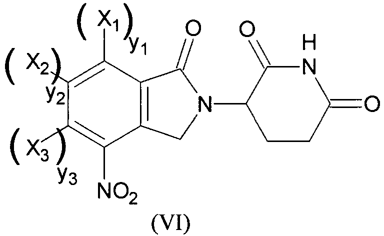 wherein X₁, X₂, X₃, y₁, y₂ and y₃ have the meaning defined above,
         - wherein when the step a) is carried out in SAG conditions, the SAG conditions are defined by the ratio of carboxylic function bearer/additive that is from 0.01 to 10, in particular 0.10,
         - wherein when the step a) is carried out in LAG conditions, the LAG conditions are defined by the parameter η that is greater than 0 mL/g and less than or equal to 1 mL/g,
         - wherein when the step a) is carried out in LAG conditions with a solid additive, the LAG conditions with a solid additive are defined:
            ➢ by the ratio of carboxylic function bearer/additive, that is from 0.01 to 10, in particular 0.10, and
            ➢ by the parameter η that is greater than 0 mL/g and less than or equal to 1 mL/g, more preferably is 0.2 mL/g,
               and
   b) a step of mechanochemical reduction of the above-mentioned compound of formula (VI), said mechanical forces being generated by twin screw extrusion, with said at least one additive, to obtain a crude product containing said compound of formula (V),
      wherein in the step b), the LAG conditions are defined by the parameter η that is greater than 0 mL/g and less than or equal to 1 mL/g.

According to a particular embodiment of the process of the invention, when the step a) is in LAG or in LAG conditions with a solid additive, the said at least one additive comprises at least one liquid additive which is dimethyl sulfoxide.

According to a particular embodiment of the process of the invention, when the step a) is in LAG or in LAG conditions with a solid additive, the said at least one additive comprises at least one liquid additive which is dimethyl isosorbide (DMI).

According to a particular embodiment of the process of the invention, in step a), the ratio of methyl 2-(bromomethyl)-3-nitrobenzoate/sodium chloride is 0.10.

According to a particular embodiment of the process of the invention, wherein in step b), the at least one liquid additive is a mixture of water and an alcohol, in particular ethanol.

As a matter of example and in a non-limiting manner, the step a) can be carried out with a mechanochemical device operating at 20-22°C, with methyl 2-(bromomethyl)-3-nitrobenzoate as the carboxylic acid bearer, 3-aminopiperidine-2,6-dione hydrochloride as the starting material, K₂CO₃ as the base, sodium chloride as solid additive and dimethyl isosorbide as liquid additive in in LAG conditions with a solid additive.

### List of figures

**Figure 1****.** ¹H NMR spectrum of compound L1 of Example 2, signal intensity (arbitrary unit) in abscissae as a function of the chemical shift in the ordinate (in ppm)
**Figure 2****.** ¹³C NMR spectrum of compound L1 of Example 2, signal intensity (arbitrary unit) in abscissae as a function of the chemical shift in the ordinate (in ppm)
**Figure 3** ¹H NMR spectrum of compound (Ia) of Example 9, signal intensity (arbitrary unit) in abscissae as a function of the chemical shift in the ordinate (in ppm)
**Figure 4****.** ¹³C NMR spectrum of compound (Ia) of Example 9, signal intensity (arbitrary unit) in abscissae as a function of the chemical shift in the ordinate (in ppm)
**Figure 5** corresponds to the detailed configuration of a ZE9 twin-screw extruder, the numbers displayed are millimeters, as follows:
   1. **LD 25:1 twin screw ((A) ; OrderNr: C04094):** A primary twin screw element with a length of 78.5 mm and a pitch of 13.5 mm, ensuring efficient conveying of materials.
   2. **Concave screw elements ((B), (E), (G), (S) ; OrderNr: C03930):** Multiple concave elements with a length of 13.5 mm and a pitch of 9 mm, placed at various intervals to aid in consistent material transport and initial mixing.
   3. **Kneading blocks:**
      **o OrderNr: ((C) ; C03935):** A kneading block with a length of 13.5 mm, 60° phase angle, and 4 kneading segments, positioned to provide initial intensive shear.
      o **OrderNr: ((F) ; C09042):** Another kneading block with a length of 13.5 mm, 90° phase angle, and 5 kneading segments, enhancing the mixing and kneading process.
      o **OrderNr: ((H), (I), (J), (L), (M), (N), (P, (Q), (R); C03291 and C03292:** Several shorter kneading elements with lengths of 3 mm, phase angles of 0° and 90°, respectively, strategically placed to continue the shearing and kneading process.
   4. **Mixing screw elements:**
      **o OrderNr: ((D) ; C04871):** A mixing element with a length of 9 mm and a pitch of 9 mm, designed to ensure thorough mixing of the reactants.
      o **OrderNr: ((K) ; C08930):** An inverted mixing element with a length of 9 mm and a pitch of 9 mm, creating backflow to enhance mixing efficiency.
   5. **Additional concave elements ((O) ; OrderNr: C03278 and C03930 :** Further concave elements with the same dimensions as previously mentioned, placed to aid in the consistent conveyance and mixing of the materials towards the end of the process.
   6. **End screws and segments:**
      **o OrderNr: C03938:** End screw elements with no pitch, ensuring complete discharge of the material from the extruder.
      o **OrderNr: ((T) ; C04872:)** End segment with a concave shape, positioned at the final stage to ensure thorough processing and discharge of the extrudate.

The present invention will be described in more detail with reference to examples below.

### EXAMPLES

### Example 1 - First example of step a) of preparation of lenalidomide by ball milling (BM)

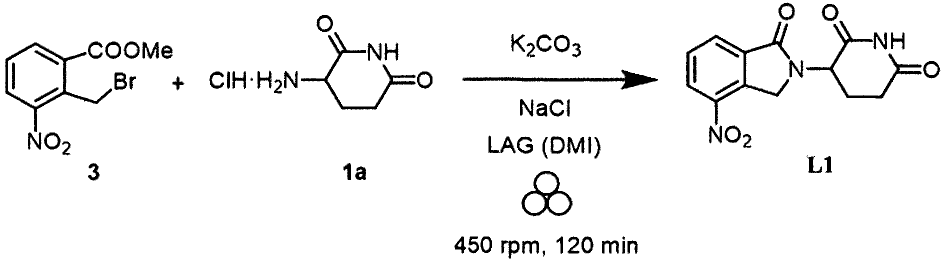

Methyl 2-(bromomethyl)-3-nitrobenzoate **(3)** (712.6 mg, 2.60 mmol, 1 eq) 3-aminopiperidine-2,6-dione hydrochloride **(1a)** (641.9 mg, 3.9 mmol, 1.5 eq), K₂CO₃ (1.435 g, 10.40 mmol, 4 eq) and NaCl (1.519 g, 26.0 mmol, 10 eq) were placed in a 20 mL hardened stainless steel jar charged with 40x5 mm hardened stainless steel balls. Then, the liquid additive was added Dimethyl-isosorbide (DMI, 862 µL, η = 0.2 µL/mg) and milled at 450 rpm for 120 min. Water (35 mL) was added to the resulting reaction crude medium, and a solid precipitate was isolated by filtration. The solid precipitate was washed with water (4x10 mL) and dried *in vacuo* in the presence of P₂O₅, to give **L1** as a grey solid (627.3 mg, 83% yield).

### Example 2 - Second example of step a) of preparation of lenalidomide by ball milling (BM)

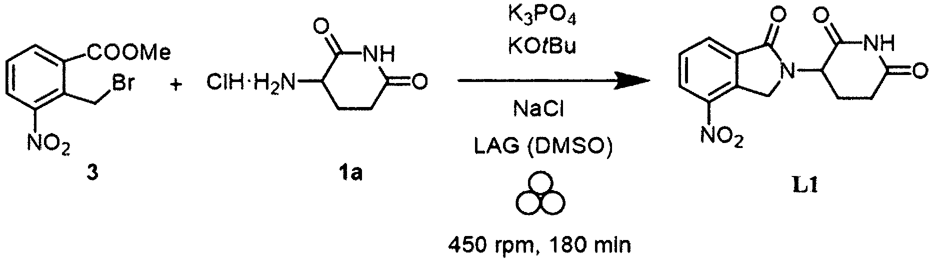

Methyl 2-(bromomethyl)-3-nitrobenzoate **(3)** (712.6 mg, 2.60 mmol) 3-aminopiperidine-2,6-dione hydrochloride **(1a)** (641.9 mg, 3.9 mmol, 1.5 eq), K₃PO₄ (1.655 g, 7.80 mmol, 3 eq), KOtBu (248.0 mg, 2.21 mmol, 0.85 eq) and NaCl (1.519 g, 26.0 mmol, 10 eq) were placed in a 20 mL hardened stainless steel jar charged with 40x5 mm hardened stainless steel balls. Then, the liquid additive was added (DMSO, 955 µL, η = 0.2 µL/mg) and milled at 450 rpm for 180 min. Water (30 mL) was added to the resulting reaction crude medium, and a solid precipitate was isolated by filtration. The solid precipitate was washed with water (3x10 mL) and dried *in vacuo* in the presence of P₂O₅, to give 3-(4-nitro-1-oxoisoindolin-2-yl) piperidine-2,6-dione **(L1)** as a blue solid (271.6 mg, 36% yield).

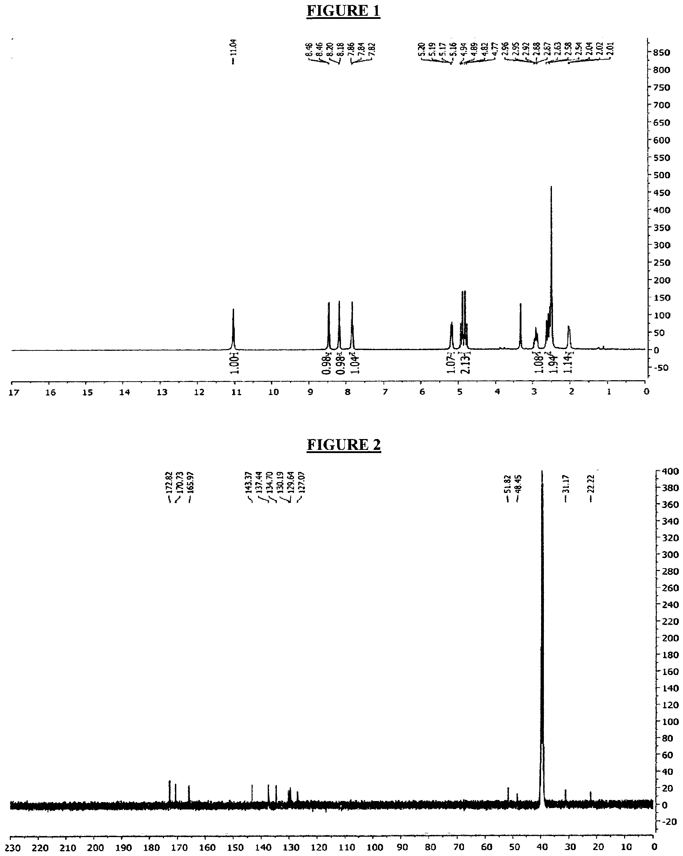

¹H NMR (400 MHz, DMSO-*d₆*, spectrum is on Figure 1) δ (ppm) 11.04 (s, 1H), 8.47 (d, ³J = 8.1 Hz, 1H), 8.19 (d, ³J = 7.4 Hz, 1H), 7.84 (t, ³J = 7.8 Hz, 1H), 5.18 (dd, ³J = 12.9, 4.8 Hz, 1H), 4.91 (d, J_{gem} = 19.3 Hz, 1H), 4.80 (d, J_{gem} = 19.3 Hz, 1H), 3.00 - 2.81 (m, 1H), 2.68 - 2.53 (m, 2H), 2.11 - 1.92 (m, 1H). ¹³C NMR (101 MHz, DMSO-d₆, spectrum is on Figure 2) δ (ppm) 172.82, 170.73, 165.97, 143.37, 137.44, 134.70, 130.19, 129.64, 127.07, 51.82, 48.45, 31.17, 22.22. HRMS ESI (+): m/z calculated for C₁₃H₁₂N₃O₅ [M+H]⁺ 290.0771, found 290.0772.

### Example 3 - Example of step b) of preparation of lenalidomide by ball milling (BM)

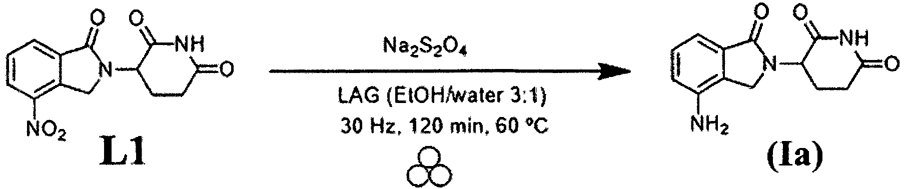

3-(4-Nitro-1-oxoisoindolin-2-yl)piperidine-2,6-dione (809.9 mg, 2.8 mmol) and Na₂S₂O₄ (1.950 g, 11.2 . mmol, 4 eq) were placed in a 25 mL stainless steel jar charged with 5x10 mm stainless steel balls. Then, liquid additive was added (EtOH, 1.380 mL; H₂O, 345 µL, η = 0.5 µL/mg) and milled at 30 Hz for 120 min at 60 °C.

After the end of the reaction, MeOH (50 mL) was added and the crude medium is filtered under vacuum. The filtrate was collected concentrated HCl (2 mL) was added and the resulting mixture was stirred for 2h, wherein a solid residue was formed. Then MeOH was evaporated and 10 mL of NaHCO₃ saturated solution were added to the solid residue to obtain a precipitate. The formed precipitate was filtered and washed further with additional 10 mL of NaHCO₃ saturated solution. Then the formed precipitate was collected under *vacuo* and dried under a desiccator to give lenalidomide (Ia) (399.2 mg, 55% calculated by qNMR (quantitative NMR) using triphenylmethane as standard) as pale-yellow solid.

### Example 4 - First example of step a) of preparation of lenalidomide by resonant acoustic mixing (RAM)

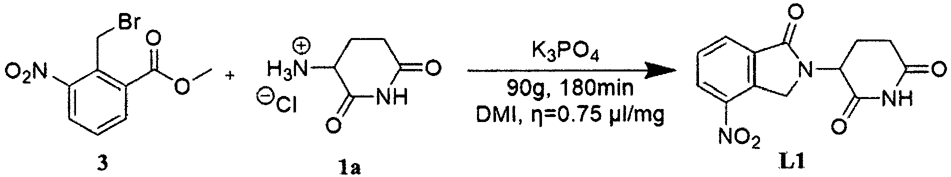

3-aminopiperidine-2,6-dione hydrochloride (495 mg, 2.92 mmol), methyl 2-(bromomethyl)-3-nitrobenzoate (400 mg, 1.46 mmol), K₃PO₄ (929.4 mg, 4.38 mmol) and dimethyl isosorbide (DMI) (1.37 ml, η = 0.75 µl/mg) were added to a 10 mL glass vial, and the content of the vial was mixed in a LabRAM II Resodyn mixer at 90 g for 180 minutes. When the mixing was finished, the resulting crude medium was transferred to a 100 mL beaker and distilled water (50 mL) was added and a precipitate was formed. Then the content of the vial was filtered under vacuum and the resulting precipitate was washed further with 50 mL of distilled water and dried *in vacuo* in the presence of P₂O₅, to give 3-(4-nitro-1-oxoisoindolin-2-yl) piperidine-2,6-dione **(L1)** (337.5 mg, 81% yield).

¹H NMR (400 MHz, DMSO-*d*₆) δ (ppm) : 11.04 (s, 1H), 8.49 (dd, *J=* 8.2, 1.0 Hz, 1H), 8.20 (dd, *J=* 7.5, 0.9 Hz, 1H), 7.86 (dd, *J =* 8.1, 7.6 Hz, 1H), 5.20 (dd, *J=* 13.2, 5.1 Hz, 1H), 4.93 (d, *J=* 19.3 Hz, 1H), 4.81 (d, *J=* 19.3 Hz, 1H), 2.93 (ddd, *J=* 16.8, 13.3, 5.2 Hz, 1H), 2.66 - 2.52 (m, 2H), 2.11 - 1.97 (m, 1H).

¹³C NMR (101 MHz, DMSO-*d*₆) δ (ppm) 173.24, 171.15, 166.39, 143.80, 137.87, 135.12, 130.61, 130.06, 127.49, 52.25, 31.60, 22.66.

HRMS ESI (+): m/z calculated for C₁₃H₁₂N₃O₅ [M+H]⁺ 290.0771, found 290.0772.

### Example 5 - Second example of step a) of preparation of lenalidomide by resonant acoustic mixing (RAM)

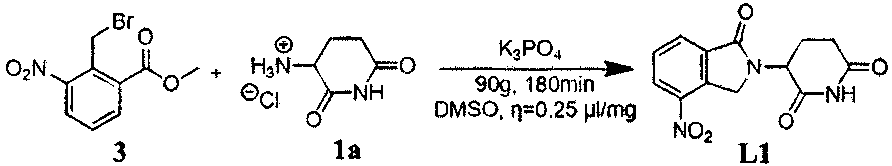

3-aminopiperidine-2,6-dione hydrochloride (495 mg, 2.92 mmol), methyl 2-(bromomethyl)-3-nitrobenzoate (400 mg, 1.46 mmol), K₃PO₄ (929.4 mg, 4.38 mmol) and DMSO (456.1 µl, *η*=0.25 µl/mg) were added to a 10 ml glass vial, and the content of the vial was mixed in a LabRAM II Resodyn mixer at 90 g for 180 minutes. When the mixing was finished, the resulting crude medium was transferred to a 100 mL beaker and distilled water (50 mL) was added and a precipitate was formed. Then the content of the vial was filtered under vacuum and the resulting precipitate was washed further with 50ml of distilled water and dried *in vacuo* in the presence of P₂O₃, to give 3-(4-nitro-1-oxoisoindolin-2-yl) piperidine-2,6-dione **(L1)** (335.7 mg, 80% yield).

¹H NMR (400 MHz, DMSO-*d*₆) δ 11.05 (s, 1H), 8.52 - 8.45 (m, 1H), 8.21 (d, *J=* 7.0 Hz, 1H), 7.86 (t, *J= 7.9* Hz, 1H), 5.20 (dd, *J=* 13.1, 5.2 Hz, 1H), 4.87 (dd, *J=* 46.2, 19.3 Hz, 2H), 2.93 (ddd, *J=* 18.5, 13.5, 5.3 Hz, 1H), 2.59 (dd, J= 23.7, 11.1 Hz, 2H), 2.10 - 2.00 (m, 1H).

¹³C NMR (101 MHz, DMSO-*d*₆) δ 173.29, 171.19, 166.44, 143.85, 137.92, 135.17, 130.11, 127.55, 52.30, 48.94, 31.65, 22.71.

HRMS ESI (+): m/z calculated for C₁₃H₁₂N₃O₅ [M+H]⁺ 290.0771, found 290.0772.

### Example 6 - Third example of step a) of preparation of lenalidomide by resonant acoustic mixing (RAM)

3-aminopiperidine-2,6-dione hydrochloride (1.26 g, 7.7 mmol), methyl 2-(bromomethyl)-3-nitrobenzoate (1.4 g, 5.1 mmol), K₂CO₃ (2.8 g, 20.4 mmol) and DMI (3.24 ml, η=0.59 µl/mg) were added to a 15 ml PTFE (polytetrafluoroethylene) jar, and the content of the jar was mixed in a LabRAM II Resodyn mixer at 90 g for 120 minutes. When the mixing was finished, the resulting crude medium was transferred to a 200 mL beaker and distilled water (100 mL) was added and a precipitate was formed. Then the content of the jar was filtered under vacuum and the resulting precipitate was washed further with 50 ml of distilled water and dried *in vacuo* in the presence of P₂O₅, to give 3-(4-nitro-1-oxoisoindolin-2-yl) piperidine-2,6-dione **(L1)** as a light brown powder (1.6 g, 75%).

### Example 7 - Fourth example of step a) of preparation of lenalidomide with K₂CO₃ and sodium chloride by resonant acoustic mixing (RAM)

3-aminopiperidine-2,6-dione hydrochloride (642.3 mg, 3.9 mmol), methyl 2-(bromomethyl)-3-nitrobenzoate (713 mg, 2.6 mmol), sodium chloride (1.52g, 26 mmol), K₂CO₃ (1.44 g, 10.4 mmol) and DMI (863 *µ*l, *η*=0.2 µl/mg) were added to a 15 ml PTFE jar, and the content of the jar was mixed in a LabRAM II Resodyn mixer at 90 g for 120 minutes. When the mixing was finished, the resulting crude medium was transferred to a 200 mL beaker and distilled water (100 mL) was added and a precipitate was formed. Then the content of the jar was filtered under vacuum and the resulting precipitate was washed further with 50 ml of distilled water and dried *in vacuo* in the presence of P₂O₅, to give 3-(4-nitro-1-oxoisoindolin-2-yl) piperidine-2,6-dione **(L1)** as a mixture (56% yield calculated by qNMR (quantitative ¹H NMR) using triphenylmethane as standard).

### Example 8 - Fifth example of step a) of preparation of lenalidomide with K₂CO₃ by resonant acoustic mixing (RAM)

3-aminopiperidine-2,6-dione hydrochloride (1.26 g, 7.7 mmol), methyl 2-(bromomethyl)-3-nitrobenzoate (1.4 g, 5.1 mmol), K₂CO₃ (2.8 g, 20.4 mmol) and DMI (1097 µl, *η* =0.2 µl/mg) were added to a 15 ml PTFE jar, and the content of the jar was mixed in a LabRAM II Resodyn mixer at 90 g for 120 minutes. When the mixing was finished, the resulting crude medium was transferred to a 200 mL beaker, and distilled water (100 mL) was added and a precipitate was formed. Then the content of the jar was filtered under vacuum and the resulting precipitate was washed further with 50ml of distilled water and dried *in vacuo* in the presence of P₂O₅, to give 3-(4-nitro-1-oxoisoindolin-2-yl) piperidine-2,6-dione **(L1)** as a light brown powder (1.1 g, 71%) (78% purity calculated by qNMR (quantitative NMR) using triphenylmethane as standard).

### Example 9 - Example of step b) of preparation of lenalidomide by resonant acoustic mixing (RAM)

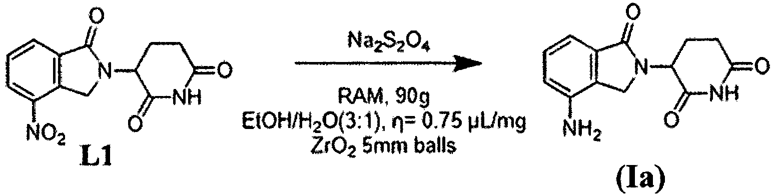

3-(4-nitro-1-oxoisoindolin-2-yl) piperidine-2,6-dione (500 mg, 1.73 mmol) and sodium dithionite (1.2 g, 6.92 mmol) were added followed by 0.96 mL of EtOH and 0.32 mL of distilled water (*η* = 0.75 µL/mg) to a 10 mL PTFE jar containing 10 x 5 mm ZrO₂ balls. Then the content of the jar was mixed in a LabRAM II Resodyn mixer at 90 g for 60 minutes. After the end of the reaction, the jar was opened to release the pressure inside and then 95 mL of MeOH were added and the crude medium was filtered under vacuum. The filtrate was collected and 5 mL of concentrated HCl were added and the mixture was stirred for 2h. Then MeOH was evaporated and 10 mL of NaHCO₃ saturated solution were added to the solid residue. The formed precipitate was filtrated and washed further with additional 10 mL of NaHCO₃ saturated solution. Then the formed precipitate was collected and dried under a desiccator to give lenalidomide (Ia) as pale-yellow solid (253.1 mg, 56%)

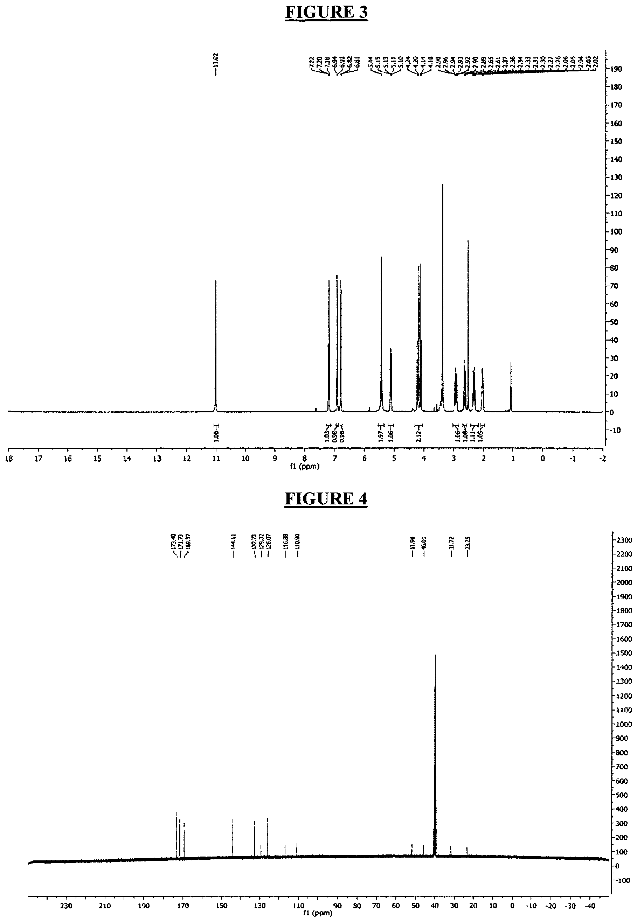

¹H NMR (400 MHz, DMSO-*d*₆) δ (ppm) 11.02 (s, 1H), 7.20 (t, *J =* 7.6 Hz, 1H), 6.93 (d, *J =* 6.9 Hz, 1H), 6.81 (d, *J = 7.8* Hz, 1H), 5.44 (s, 2H), 5.12 (dd, *J =* 13.3, 5.1 Hz, 1H), 4.17 (dd, *J =* 40.6, 17.0 Hz, 2H), 3.02 - 2.85 (m, 1H), 2.63 (d, *J =* 16.1 Hz, 1H), 2.32 (qd, *J =* 13.2, 4.5 Hz, 1H), 2.10 - 1.97 (m, 1H)-(Figure 3)

¹³C NMR (101 MHz, DMSO-*d*₆) δ (ppm) 173.40, 171.73, 169.37, 144.11, 132.73, 129.32, 126.07, 116.88, 110.90, 51.98, 46.01, 31.72, 23.25. (Figure 4)

HRMS ESI (+): m/z calculated for C₁₃H₁₄N₃O₃ [M+H]⁺ 260.1030, found 260.1031.

### Example 10 - Step a) of preparation of lenalidomide under twin screw extrusion (TSE)

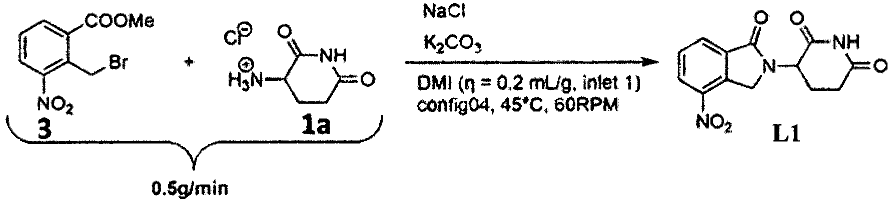

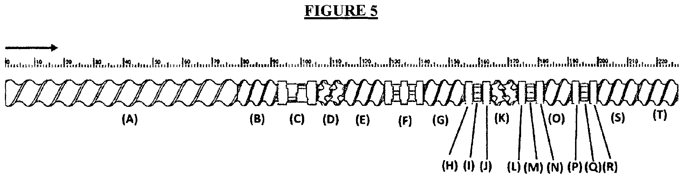

Solid methyl 2-(bromomethyl)-3-nitrobenzoate (2.00 g, 7.30 mmol, 1 eq), 3-aminopiperidine-2,6-dione hydrochloride (1.80 mg, 10.95 mmol, 1.50 eq), K₂CO₃ (4.03 g, 29.19 mmol, 4.00 eq) and NaCl (4.26 g, 72.97 mmol, 10.00 eq) were homogenized within a mortar and pestle and then transferred inside one closed feeder put under nitrogen. The screw profile used is described in Figure 5. Subsequently the 4 segments of the ZE9 barrel (Three-Tec, diameter: 9 mm; L/D = 25:1) were heated at 45°C. Once the temperature was reached and stabilized, extrusion started at 60 RPM. Solid starting materials were fed at approximately 0.5 g/min while dimethyl isosorbide serving as liquid additive entered at 0.1 mL/min (*η* = 0.2 mL/g, liquid inlet located at 34.5 mm from the solid addition port or 73 mm from the beginning of the screw). After about 15 minutes 30 seconds, extrudate began emerging as one pale white solid labeled F1. That initial fraction was collected for approximately 8 minutes and 30 seconds yielding mF1 = 170 mg with ¹H NMR analysis revealing complete conversion of methyl 2-(bromomethyl)-3-nitrobenzoate, the presence of 38% of 3-(4-nitro-1-oxoisoindolin-2-yl) piperidine-2,6-dione with 62% alkylated intermediates.

Another subsequent fraction F2 emerged as one greyish paste for roughly one minute yielding mF2 = 680 mg with ¹H NMR showing full conversion of methyl 2-(bromomethyl)-3-nitrobenzoate and 3-aminopiperidine-2,6-dione hydrochloride though numerous intermediates remained observable. At this point, the entire mixture previously held inside the feeder was depleted therefore F1 was reintroduced inside the feeder. Subsequently a third fraction F3 appeared gradually as some grey powder. Once the feeder ran completely empty, F2 addition followed and after that point NaCl at about 7.6 g was introduced inside the feeder. After 9 minutes, F3 collection stopped, yielding mF3 = 2.20 g, and purge fraction processing was initiated. Approximately 8 minutes 30 seconds afterward, another 3 g portion of NaCl entered feeder. About 7 minutes after that moment, extrudate changed appearance from greyish powder toward faint greyish powder and another 3 g portion of NaCl was loaded inside the feeder to continue the purge. Finally, the extrusion process was stopped 11 minutes later (mFpurge = 11.45g).

At the end of the extrusion, 2 fractions were collected: F3 and Fpurge.

Fpurge corresponds to the fraction of products when NaCl is filled into the feeder to progressively flush the remaining mixture that is inside the barrel, and the barrel is emptied. Fpurge consists in NaCl, 3-(4-nitro-1-oxoisoindolin-2-yl) piperidine-2,6-dione and residual methyl 2-(bromomethyl)-3-nitrobenzoate and 3-aminopiperidine-2,6-dione hydrochloride, or by-products that remain in the extruder after the cycloamidation has ceased.

To the resulting F3 reaction crude, water (8 mL) was added and the precipitate was isolated by filtration under vacuum. The solid was washed with water until the pH became neutral (3x10 mL) and air dried for 20 min before drying *in vacuo* at 45°C (vacuum oven) overnight to give 3-(4-nitro-1-oxoisoindolin-2-yl) piperidine-2,6-dione **(L1)** as pale whitish solid (240.6 mg, 97% pure according to qNMR done with triphenylmethane as standard). To the resulting F4 reaction crude, water (25 mL) was added and the precipitate was isolated by filtration under vacuum. The solid was washed with water (3x10 mL) and air dried for 20 min before drying *in vacuo* at 45°C (vacuum oven) overnight to give 3-(4-nitro-1-oxoisoindolin-2-yl) piperidine-2,6-dione **(L1)** as a white (slightly grey) solid (1.2710 g, 89% pure according to qNMR done with triphenylmethane as standard). For further purification, Fpurge was washed with cyclopentyl methyl ether (CPME) (2x10mL), filtered under vacuum and dried *in vacuo* at 45°C (vacuum oven) overnight to give 3-(4-nitro-1-oxoisoindolin-2-yl) piperidine-2,6-dione **(L1)** as a white solid (1.0126 g, 97% pure according to qNMR done with triphenylmethane).

Finally, the addition of the mass of product by combining F3 and Fpurge allows to calculate the following yield : 0.97*(0.2406+1.0126) / 2.11 = 62%.

### Space-Time Yield (STY) calculations:

**F3** was collected in 9min, the pure mass of 3-(4-nitro-1-oxoisoindolin-2-yl) piperidine-2,6-dione **(L1)** obtained was 0.97*240.6 = 233.4 mg and the free volume of the reactor (ZE9 barrel) is 12416mm³ so the STY is 3005 kg.m⁻³.day⁻¹.

### Example 11 - Step b) of preparation of lenalidomide under twin screw extrusion (TSE)

3-(4-nitro-1-oxoisoindolin-2-yl) piperidine-2,6-dione (2.00 g, 6.92 mmol) and sodium dithionite (4.8 g, 27.68 mmol) is homogenized within a mortar and pestle and then transferred inside one closed feeder. A mixture of EtOH/H₂O 3:1 (volumetric ratio) is prepared and charged into a syringe connected to a syringe pump. The barrel of the extruder (ZE9, LD:25:1, Three-Tec) is heated at 70°C. When the barrel is at the target temperature, 3-(4-nitro-1-oxoisoindolin-2-yl) piperidine-2,6-dione and sodium dithionite are introduced into the extruder at 0.5 g/min and simultaneously the mixture of EtOH/H₂O is introduced at the second liquid introduction port at 0.375 mL/min (LAG h = 0.75 µL/mg). All the extrudate that goes out of the extruder is purge until the extrudate becomes white. The white extrudate is collected in a tube containing 50 mL of MeOH. After the end of extrusion, the mixture of extrudate and ethyl acetate was transferred in a filtration funnel and washed further with 10 mL of MeOH. The filtrate is collected and 10 mL of concentrated HCl are added and the mixture is stirred for 2h. Then MeOH is evaporated and 10 mL of NaHCO₃ saturated solution are added to the solid residue. The formed precipitate is filtrated and washed further with 10 more mL of NaHCO₃ solution. Then the formed precipitate is collected and dried under a desiccator to give lenalidomide (Ia) as pale-yellow solid.

## Claims

1. Use of a mechanical force to generate a mechanochemical reaction in the implementation of a process of preparation in a reactor of a compound of formula (I): 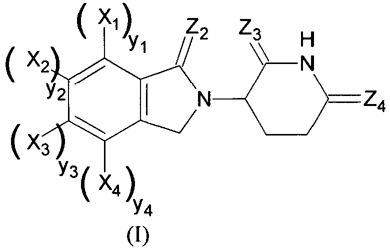 wherein:
- Z₂, Z₃ and Z₄, identical or different, are chosen among: oxygen or sulfur atom,
- X₁, X₂, X₃ and X₄, identical or different, are chosen among: (C₁-C₅)alkyl group, (C₁-C₅)alkoxy group, amino group, halogen atom, trifluoromethyl group, hydroxy group, (H or C₁-C₁₀)acyl group, a 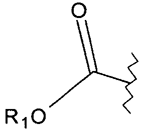 group, or a 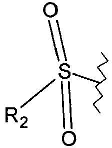 group
wherein R₁ is chosen among: hydrogen atom, (C₁-C₅)alkyl group, or -NR₁ₐR_{1b} group, R₁ₐ and R_{1b}, identical or different, are chosen among: hydrogen atom or (C₁-C₅)alkyl group,
and R₂ is chosen among: hydroxy group, (C₁-C₅)alkoxy group, -NR₂ₐR_{2b},
R₂ₐ and R_{2b}, identical or different, are chosen among: hydrogen atom or (C₁-C₅)alkyl group
- y₁, y₂, y₃ and y₄, identical or different, are equal to 0 or 1, and provided that y₁ + y₂ + y₃ + y₄ is 1 or 2,
- and provided that at least one X₁, X₂, X₃ and X₄ is an amino group,
or a pharmaceutically acceptable salt thereof,
said process comprising 2 steps, using 3-aminopiperidine-2,6-dione hydrochloride as starting material, wherein at least one step is mechanochemical.

2. The use, according to claim 1, for the preparation of the compound of formula (I), wherein y₁ = y₂ = y₃ = 0, y₄ = 1, Z₂, Z₃ and Z₄ are oxygen atoms and X₄ is an amino group, and corresponding to the following formula (Ia):

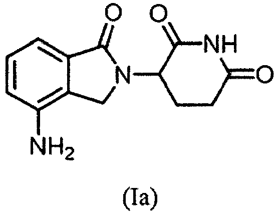

3. The use, according to any one of claims 1 or 2, wherein said process is carried out in batch or in flow.

4. The use according to any one of claims 1 to 3, wherein the mechanical forces are generated by a method chosen among: ball-milling (BM), in particular vibrating ball-milling (VBM) or planetary ball-milling (PBM), resonant acoustic mixing (RAM) or screw extrusion (single, twin or multi).

5. The use, according to any one of claims 1 to 4, wherein at least one step is mechanochemical, in particular at least two steps are mechanochemical.

6. The use according to any one of claims 1 to 5, wherein said process of preparation provides at least 3.10³ kg.m⁻³ of said reactor.day⁻¹, in particular from 3.10³ kg.m⁻³ of said reactor.day⁻¹ to 68 000 kg.m⁻³ of said reactor.day⁻¹, of said compound of formula (I) or of said pharmaceutically acceptable salt thereof.

7. A mechanochemical process of preparation of a compound of formula (I), 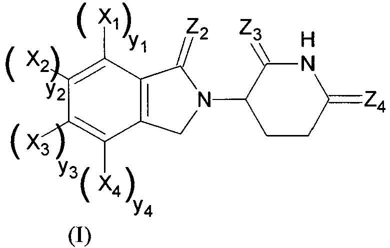 wherein:
- Z₂, Z₃ and Z₄, identical or different, are chosen among: oxygen or sulfur atom,
- X₁, X₂, X₃ and X₄, identical or different, are chosen among: (C₁-C₅)alkyl group, (C₁-C₅)alkoxy group, amino group, halogen atom, trifluoromethyl group, hydroxy group, (H or C₁-C₁₀)acyl group, a 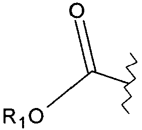 group, or a 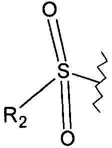 group
wherein R₁ is chosen among: hydrogen atom, (C₁-C₅)alkyl group, or -NR₁ₐR_{1b} group, R₁ₐ and R_{1b}, identical or different, are chosen among: hydrogen atom or (C₁-C₅)alkyl group,
and R₂ is chosen among: hydroxy group, (C₁-C₅)alkoxy group, -NR₂ₐR_{2b},
R₂ₐ and R_{2b}, identical or different, are chosen among: hydrogen atom or (C₁-C₅)alkyl group
- y₁, y₂, y₃ and y₄, identical or different, are equal to 0 or 1, and provided that y₁ + y₂ + y₃ + y₄ is 1 or 2,
- and provided that at least one X₁, X₂, X₃ and X₄ is an amino group,
said process comprising:
a) a cycloamidation step, using 3-aminopiperidine-2,6-dione hydrochloride as starting material, and a carboxylic function bearer, said carboxylic function bearer being in particular methyl 2-(bromomethyl)-3-nitrobenzoate,
a base in particular chosen among tripotassium phosphate and potassium carbonate, and optionally at least one additive, in particular said at least one additive being sodium chloride, said process using mechanochemical forces, and
being carried out under neat grinding, or in solid-assisted grinding (SAG) conditions, or in liquid-assisted grinding (LAG) conditions, or in LAG conditions with a solid additive,
to obtain a crude product containing a compound of formula (II): 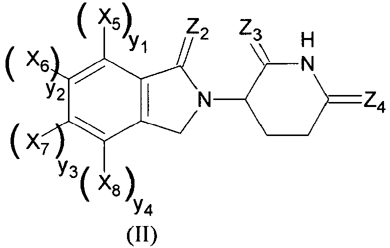
wherein:
- Z₂, Z₃ and Z₄ having the meaning defined above,
- X₅, X₆, X₇ and X₈, identical or different, are chosen among: (C₁-C₅)alkyl group, (C₁-C₅)alkoxy group, nitro group, halogen atom, trifluoromethyl group, hydroxy group, (H or C₁-C₁₀)acyl group, a 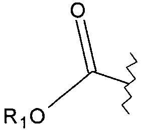 group, or a 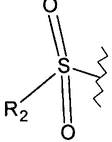 group
wherein R₁ is chosen among: hydrogen atom, (C₁-C₅)alkyl group, or -NR₁ₐR_{1b} group, R₁ₐ and R_{1b}, identical or different, are chosen among: hydrogen atom or (C₁-C₅)alkyl group,
and R₂ is chosen among: hydroxy group, (C₁-C₅)alkoxy group, -NR₂ₐR_{2b},
R₂ₐ and R_{2b}, identical or different, are chosen among: hydrogen atom or (C₁-C₅)alkyl group
- y₅, y₆, y₇ and y₈, identical or different, are equal to 0 or 1, and provided that y₅ + y₆ + y₇ + y₈ is 1 or 2,
- and provided that at least one X₅, X₆, X₇ and X₈ is a nitro group,
and,
b) a step of mechanochemical reduction of the above-mentioned compound of formula (II), with at least one additive, using mechanochemical forces, in liquid-assisted grinding (LAG) conditions with sodium dithionite, to obtain a crude product containing said compound of formula (I).

8. The mechanochemical process, according to claim 7, wherein the step a) is carried out with at least one additive.

9. The mechanochemical process, according to any one of claims 7 or 8, wherein the step a) is carried out with at least one additive, and:
• when said at least one additive is solid under mechanical forces, the step a) is carried out in solid-assisted grinding (SAG) conditions,
or
• when said at least one additive is liquid under mechanical forces, the step a) is carried out in liquid-assisted grinding (LAG) conditions,
or
• when said at least one additive comprises a solid additive and a liquid additive under mechanical forces, the step a) is carried out in LAG conditions with a solid additive.

10. The mechanochemical process, according to any one of claims 7 to 9, wherein in step a) and/or in step b), the mechanical forces are generated by a method chosen among: ball-milling (BM), in particular vibrating ball-milling (VBM), planetary ball-milling (PBM), resonant acoustic mixing (RAM) or screw extrusion (single, twin or multi).

11. The mechanochemical process, according to any one of claims 7 to 10, wherein when the mechanical forces are generated by RAM, said mechanochemical process is carried out in a vessel wherein a m x g force is applied, m being a positive number, in particular from about 2 g to 100g, in particular 90 g.

12. The mechanochemical process, according to any one of claims 7 to 11, for the preparation of a compound of formula (V): 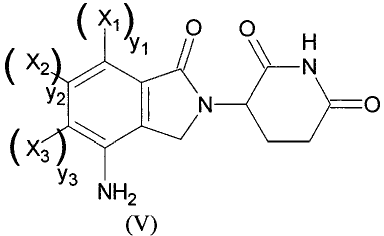
wherein X₁, X₂, X₃, y₁, y₂ and y₃ have the meaning defined above,
in particular of formula (Ia) 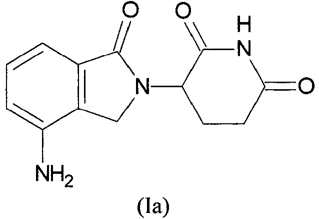
said process comprising:
a) a cycloamidation step, wherein 3-aminopiperidine-2,6-dione hydrochloride is used as starting material
said carboxylic function bearer is methyl 2-(bromomethyl)-3-nitrobenzoate,
and said base is in particular K₂CO₃
and said at least one additive is, in particular sodium chloride, dimethyl sulfoxide or dimethyl isosorbide, or a mixture of thereof,
and said mechanical forces are generated by BM and are carried out in SAG conditions, or in LAG conditions or in LAG conditions with a solid additive,
to obtain a crude product containing a compound of formula (VI), 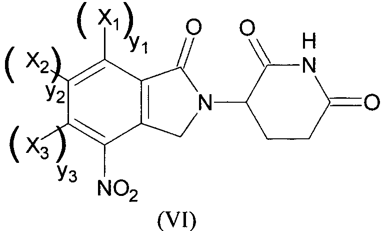
wherein X₁, X₂, X₃, y₁, y₂ and y₃ have the meaning defined above,
• wherein when the step a) is carried out in SAG conditions, the SAG conditions are defined by the ratio of carboxylic function bearer/additive that is from 0.01 to 10, in particular 0.10,
• wherein when the step a) is carried out in LAG conditions, the LAG conditions are defined by the parameter η that is greater than 0 µL/mg and less than or equal to 1 µL/mg, more preferably is comprised from about 0.05 µL/mg to 0.35 µL/mg, more preferably 0.2 µL/mg,
• wherein when the step a) is carried out in LAG conditions with a solid additive, the in LAG conditions with a solid additive are defined :
➢ by the ratio of carboxylic function bearer/additive, that is from 0.01 to 10, in particular 0.10, and
➢ by the parameter η that is greater than 0 µL/mg and less than or equal to 1 µL/mg, more preferably is comprised from about 0.05 µL/mg to 0.35 µL/mg, more preferably 0.2 µL/mg,
in particular, said compound of formula (VI) being recovered from the crude product containing it, by a washing with water, by a filtration and by a removal of said water.
and
b) a step of mechanochemical reduction of the above-mentioned compound of formula (VI), said mechanical forces being generated by BM, with said at least one additive, to obtain a crude product containing said compound of formula (V),
wherein in the step b), the LAG conditions are defined by the parameter η that is greater than 0 µL/mg and less than or equal to 1 µL/mg, preferably is comprised from about 0.25 µL/mg to 0.75 µL/mg, more preferably 0.5 µL/mg,
in particular, said compound of formula (V) being recovered from the crude product containing it, by a washing with boiling solvent, in particular acetone, a filtration and an evaporation of said solvent.

13. The mechanochemical process, according to any one of claims 7 to 11, for the preparation of a compound of formula (V): 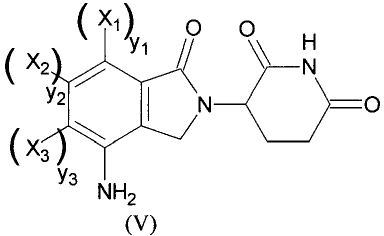
wherein X₁, X₂, X₃, y₁, y₂ and y₃ have the meaning defined above,
in particular of formula (Ia) 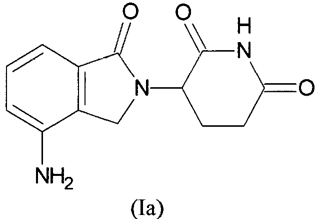
said process comprising:
a) a cycloamidation step, wherein 3-aminopiperidine-2,6-dione hydrochloride is used as starting material
said carboxylic function bearer is methyl 2-(bromomethyl)-3-nitrobenzoate and said base is in particular K₃PO₄ or K₂CO₃,
and said at least one additive is used,
and said mechanical forces are generated by RAM and are carried out in LAG conditions or in LAG conditions with a solid additive, in particular sodium chloride,
to obtain a crude product containing a compound of formula (VI), 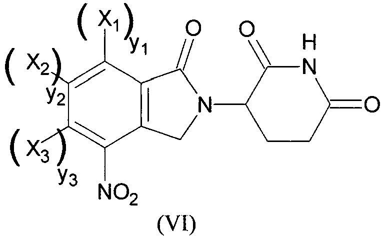
wherein X₁, X₂, X₃, y₁, y₂ and y₃ have the meaning defined above,
• wherein when the step a) is carried out in LAG conditions, the LAG conditions are defined by the parameter η that is greater than 0 µL/mg and less than or equal to 1 µL/mg, preferably is comprised from about 0.25 µL/mg to 0.75 µL/mg, more preferably 0.25 µL/mg,
or
• wherein when the step a) is carried out in LAG conditions with a solid additive, the LAG conditions with a solid additive are defined :
➢ by the ratio of carboxylic function bearer/additive, that is from 0.01 to 10, in particular 0.10, and
➢ by the parameter η that is greater than 0 µL/mg and less than or equal to 1 µL/mg, more preferably is comprised from about 0.05 µL/mg to 1 µL/mg, more preferably 0.2 µL/mg,
in particular said the compound of formula (VI) being recovered from the crude product containing it, by a washing with water, a filtration and a removal of said water,
and
b) a step of mechanochemical reduction of the above-mentioned compound of formula (VI), said mechanical forces being generated by RAM, with said at least one additive, to obtain a crude product containing said compound of formula (V),
wherein in the step b), the LAG conditions are defined by the parameter η that is greater than 0 µL/mg and less than or equal to 1 µL/mg, preferably is comprised from about 0.60 µL/mg to 0.90 µL/mg, more preferably 0.75 µL/mg.

14. The mechanochemical process according to claim 13, wherein the compound of formula (V) is recovered from the crude product containing it, by a first filtration to obtain a filtrate, placing said filtrate in contact with an aqueous solution of hydrochloric acid, a removal of said solution of hydrochloric acid, placing in contact with an aqueous solution of sodium hydrogencarbonate said filtrate, a second filtration to obtain a second filtrate, and a removal of said aqueous solution of sodium hydrogencarbonate from the second filtrate.

15. The mechanochemical process, according to any one of claims 7 to 11, for the preparation of a compound of formula (V): 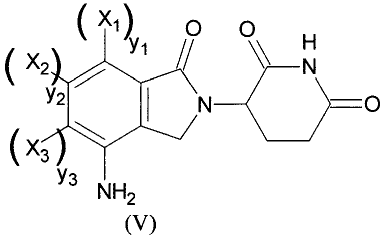
wherein X₁, X₂, X₃, y₁, y₂ and y₃ have the meaning defined above,
in particular of formula (Ia) 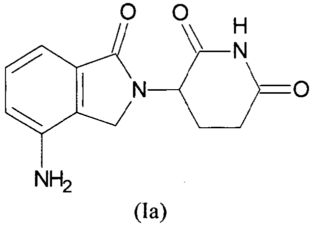
said process comprising:
a) a cycloamidation step, wherein 3-aminopiperidine-2,6-dione hydrochloride is used as starting material,
said carboxylic function bearer is methyl 2-(bromomethyl)-3-nitrobenzoate,
and said base is in particular K₂CO₃,
and said at least one additive is in particular sodium chloride, dimethyl sulfoxide, or dimethyl isosorbide, or a mixture of thereof,
and said mechanical forces are generated by twin screw extrusion, and are carried out in SAG, or LAG or in LAG conditions with a solid additive,
to obtain a crude product containing a compound of formula (VI), 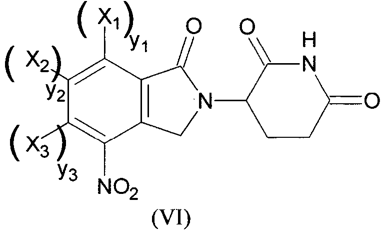
wherein X₁, X₂, X₃, y₁, y₂ and y₃ have the meaning defined above,
• wherein when the step a) is carried out in SAG conditions, the SAG conditions are defined by the ratio of carboxylic function bearer/additive that is from 0.01 to 10, in particular 0.10,
• wherein when the step a) is carried out in LAG conditions, the LAG conditions are defined by the parameter η that is greater than 0 mL/g and less than or equal to 1 mL/g,
• wherein when the step a) is carried out in LAG conditions with a solid additive, the LAG conditions with a solid additive are defined:
➢ by the ratio of carboxylic function bearer/additive, that is from 0.01 to 10, in particular 0.10, and
➢ by the parameter η that is greater than 0 mL/g and less than or equal to 1 mL/g, more preferably is 0.2 mL/g,
and
b) a step of mechanochemical reduction of the above-mentioned compound of formula (VI), said mechanical forces being generated by twin screw extrusion, with said at least one additive, to obtain a crude product containing said compound of formula (V),
wherein in the step b), the LAG conditions are defined by the parameter η that is greater than 0 mL/g and less than or equal to 1 mL/g.
